# EUROPEAN PATENT APPLICATION

(11) **EP 4 210 068 A1**
(43) Date of publication of application: **12.07.2023**
(21) Application number: 23150276.6
(22) Date of filing: 04.01.2023
(51) Int. Cl.: G16H 30/00

(54) **MEDICAL INFORMATION PROCESSING SYSTEM, MEDICAL INFORMATION PROCESSING METHOD, AND PROGRAM**

(30) Priority: 07.01.2022 JP 2022001733; 16.12.2022 JP 2022201458
(71) Applicant: Canon Medical Systems Corporation, Tochigi 324-0036 (JP)
(72) Inventor: SHIROISHI, Ryo, Otawara-shi, 324-0036 (JP)
(74) Representative: Marks & Clerk LLP

(57) **Abstract**

A medical information processing system according to an embodiment includes an acquisition unit, an identification unit, an image generation unit, and a transmission unit. The acquisition unit acquires information on an examination target. The identification unit identifies a reference image corresponding to the examination target on the basis of the information on the examination target. The image generation unit generates an edited image generated by editing the reference image. The transmission unit transmits order information to which the edited image has been attached.

## Description

### FIELD

Embodiments disclosed in the specification and drawings relate to a medical information processing system, a medical information processing method, and a program.

### BACKGROUND

When a medical doctor orders an image examination from a radiology department, for example, order information including instructions generated by the medical doctor and indicated by text is sent from the medical department to the radiology department via an ordering system. At the time of ordering an image examination, if instructions from a medical doctor are not accurately delivered, unnecessary examinations may be performed or re-examination may be required.

This causes disadvantages such as unnecessary radiation exposure to patients (subjects) and wasteful use of medical resources. In particular, since spatial information (a range of an imaging target and a direction of imaging) and information on the appearance of an image (a target to be viewed, a target that need not be viewed, a degree of accuracy of an image, and a final image) are difficult to verbalize, it may be difficult to deliver instructions. Therefore, generated order information may be accompanied by a schema, for example, to assist with understanding of the instructions.

Schemas are often created by hand, for example, by medical doctors. For this reason, medical doctors are required to have drawing ability and to take the effort to draw schemas. Furthermore, in cases where a medical doctor does not draw a schema or has poor drawing ability, and the like, it may be difficult to accurately deliver detailed instructions to the radiology department.

### SUMMARY

An object of embodiments disclosed in the specification and drawings is to allow instructions regarding an examination to be able to be accurately and easily delivered. However, the object of the embodiments disclosed in the specification and drawings is not limited to the above object. An object corresponding to each effect of each configuration shown in embodiments which will be described later can be positioned as another object.
(1) A medical information processing system according to an embodiment includes an acquisition unit, an identification unit, an image generation unit, and a transmission unit. The acquisition unit acquires information on an examination target. The identification unit identifies a reference image corresponding to the examination target on the basis of the information on the examination target. The image generation unit generates an edited image generated by editing the reference image. The transmission unit transmits order information to which the edited image has been attached.
(2) In the medical information processing system according to the above (1), the acquisition unit further acquires patient information on a target patient, and the identification unit identifies the reference image on the basis of the information on the examination target and the patient information.
(3) In the medical information processing system according to the above (1), the identification unit identifies the reference image from among previously captured images of the examination target.
(4) In the medical information processing system according to the above (2), the reference image is identified from among past self-captured images of the examination target in the target patient.
(5) In the medical information processing system according to the above (2) or (4), the identification unit identifies the reference image according to characteristics of the target patient included in the patient information.
(6) In the medical information processing system according to the above (2) or (4), the identification unit identifies the reference image from among model images expressing a human body structure according to the characteristics of the target patient.
(7) In the medical information processing system according to the above (5) or (6), the characteristics of the target patient include at least one of age, sex, physique, or diseases.
(8) In the medical information processing system according to any one of the above (1) to (7), the identification unit identifies the reference image on the basis of selection information transmitted through an input interface operated by an operator.
(9) In the medical information processing system according to any one of the above (1) to (8), the image generation unit generates the edited image by changing an imaging direction of the reference image.
(10) In the medical information processing system according to any one of the above (1) to (9), the image generation unit generates the edited image obtained by changing an imaging range of the reference image.
(11) In the medical information processing system according to any one of the above (1) to (10), the image generation unit generates the edited image by applying an image filter to the reference image.
(12) In the medical information processing system according to any one of the above (1) to (11), the image generation unit generates the edited image obtained by removing an area of a part of the imaging range of the reference image.
(13) The medical information processing system according to any one of the above (1) to (12) further includes a history information setting unit configured to set history information indicating a history of operations performed on the reference image. The transmission unit transmits the order information to which the edited image and the history information have been attached.
(14) The medical information processing system according to any one of the above (1) to (13) further includes an explanatory information setting unit configured to set explanatory information for the edited image. The transmission unit transmits the order information to which the edited image for which the explanatory information has been set has been attached.
(15) In the medical information processing system according to any one of the above (1) to (14), the image generation unit generates determine a target portion based on text information in examination purpose information, detect the target portion in the reference image, and generate the edited image based on the detected target portion.
(16) In the medical information processing system according to the above (15), the image generation unit generates the edited image by changing a display range of the reference image so that the entire of the target portion is included in the display range and the display area of the target portion is maximized or the target portion in the reference image is enlarged.
(17) In the medical information processing system according to the above (15), the image generation unit generates the edited image by changing a display direction of the reference image so that the display area of the target portion is maximized or the edge amount of the target portion is maximized.
(18) In the medical information processing system according to the above (15), the image generation unit generates the edited image by deleting a region other than the target portion or deleting portions other than any portions related to the target portion and a target disease.
(19) In the medical information processing system according to the above (15), the image generation unit generates the edited image by changing an image filter based on combination of the text information indicating the examination purpose and the target portion.
(20) A medical information processing method, performed by a computer, according to an embodiment includes acquiring information on an examination target, identifying a reference image corresponding to the examination target on the basis of the information on the examination target, generating an edited image generated by editing the reference image, and transmitting order information to which the edited image has been attached.
(21) A program according to an embodiment causes a computer to acquire information on an examination target, identify a reference image corresponding to the examination target on the basis of the information on the examination target, generate an edited image generated by editing the reference image, and transmit order information to which the edited image has been attached.

### BRIEF DESCRIPTION OF THE DRAWINGS

FIG. 1 is a block diagram showing an example of a configuration of an intra-hospital system 1 of a first embodiment.
FIG. 2 is a block diagram showing an example of a configuration of a medical information processing system 100 of the first embodiment.
FIG. 3 is a flowchart showing an example of processing in the medical information processing system 100 of the first embodiment.
FIG. 4 is a flowchart showing an example of processing in the medical information processing system 100 of the first embodiment.
FIG. 5 is a diagram showing an example of a reference image GA10 and an edited image GA11.
FIG. 6 is a diagram showing an example of a reference image GA20 and a first edited image GA21 to a fourth edited image GA24.
FIG. 7 is a diagram showing an example of a reference image GA30 and a first edited image GA31 to a third edited image GA33.
FIG. 8 is a diagram showing an example of a reference image GA40 and an edited image GA41.
FIG. 9 is a diagram showing an example of a reference image GA50, a first edited image GA51, and a second edited image GA52.
FIG. 10 is a diagram showing an example of a reference image GA60 and an edited image GA61.
FIG. 11 is a block diagram showing an example of a configuration of a medical information processing system 200 of a second embodiment.
FIG. 12 is a flowchart showing an example of processing in the medical information processing system 200 of the second embodiment.
FIG. 13 is a diagram showing a state in which explanatory information has been added to the third edited image GA33.

### DETAILED DESCRIPTION

Hereinafter, a medical information processing system, a medical information processing method, and a program according to embodiments will be described with reference to the drawings.

### <First embodiment>

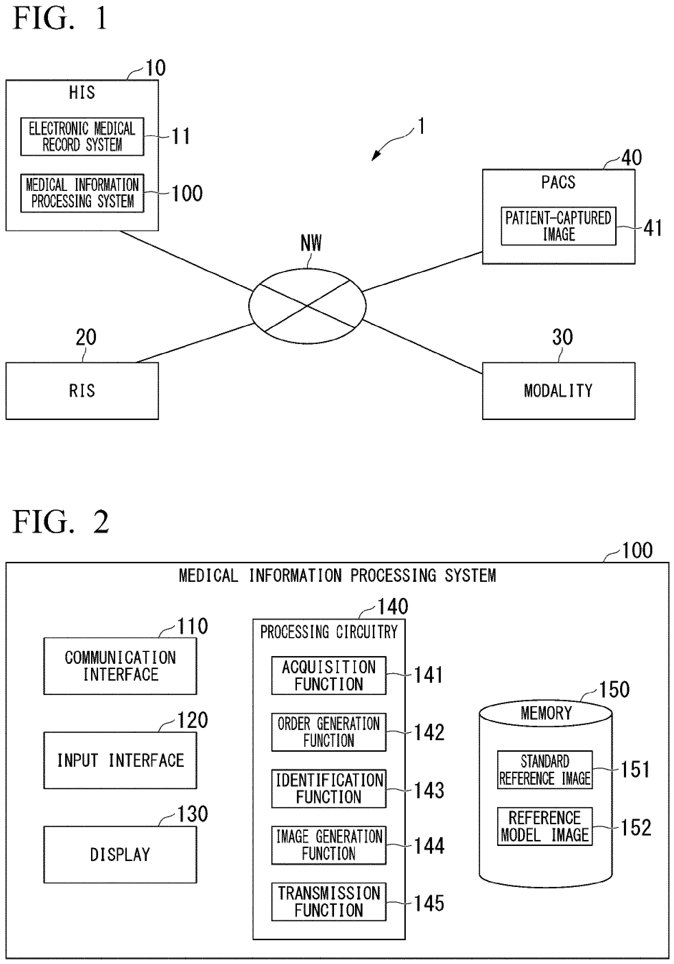

FIG. 1 is a block diagram showing an example of a configuration of an intra-hospital system 1 of the first embodiment. The intra-hospital system 1 of the first embodiment includes, for example, a hospital information system (hereinafter referred to as an HIS) 10, a radiology information system (hereinafter referred to as an RIS) 20, a medical image diagnostic device (modality) 30, and a picture archiving and communication system (PACS) 40. The HIS 10 includes a medical information processing system 100.

The HIS 10 is a computer system that supports work within a hospital. Specifically, the HIS 10 has various subsystems including an electronic medical record system 11 and the medical information processing system 100. The various subsystems include, for example, a medical accounting system, a medical appointment system, a hospital visit reception system, and a hospitalization/discharging management system.

The HIS 10 includes, for example, a computer such as a server device or a client terminal including a processor such as a central processing unit (CPU), memories such as a read only memory (ROM) and a random access memory (RAM), a display, an input interface, and a communication interface.

A medical professional such as a medical doctor (hereinafter referred to as a medical doctor or the like) inputs or refers to various types of information about patients (hereinafter referred to as patient information) using the electronic medical record system 11 in the HIS 10. Patient information of each patient is managed by being associated with a patient ID by which each patient can be identified.

The electronic medical record system 11 stores electronic medical records of a plurality of patients. Electronic medical records contain various types of information about patients, including patient information. Patient information includes information indicating characteristics of patients. Characteristics of a patient include, for example, the age, sex, physique (height, weight, etc.), suspected disease, medical history, and the like of the patient. Information on the age, sex, and physique of a patient is useful information by which the structure of the body of a target patient can be estimated from the physical characteristics of the target patient. Information on a suspected disease name and medical history of a patient is useful information by which the structure of the body of a target patient that is different from that in a healthy state can be estimated.

A medical doctor or the like issues an image examination order by applying generation instruction information to the medical information processing system 100 in the HIS 10. The HIS 10 transfers order information corresponding to the image examination order to other systems such as the RIS 20. The medical information processing system 100 serves as an ordering system. The generation instruction information includes, for example, information indicating the content of the image examination order.

The ordering system is a system that transmits instructions (orders) for things such as examinations and prescriptions to the relative department. Orders include not only image examination instructions but also physiological examination instructions, laboratory test instructions, prescription drug instructions, dietary maintenance instructions, and the like. In image examinations, a medical department generally issues an image examination order and instructs a radiology department to perform the examination.

The HIS 10 starts generation of order information by the medical information processing system 100 when a medical doctor or the like acquires generation instruction information for issuing an image examination order. Before the medical information processing system 100 generates the order information, the HIS 10 causes the electronic medical record system 11 to transmit stored patient information of a patient who is an examination target to the medical information processing system 100. The examination target relates to the body of the patient to be examined, and may be, for example, a body part such as an organ in which a disease has occurred, or a designated part regardless of the relationship with the disease. Alternatively, the examination target may be the entire body or may extend throughout the body, such as nerves and blood vessels.

The medical information processing system 100 generates the order information on the basis of generation instruction information, patient information, and other information about the examination target. The medical information processing system 100 transmits the generated order information to the RIS 20 along with the patient information. The ordering system may issue a prescription order or the like for a pharmacy department system which is not shown.

The RIS 20 is a computer system that supports works in an image diagnostic department. The RIS 20 performs cooperation of reservation information with examination equipment, management of examination information, and the like in addition to reservation management of image examination orders in cooperation with the HIS 10. The RIS 20 includes, for example, a computer such as a server device or a client terminal including a processor such as a CPU, memories such as a ROM and a RAM, a display, an input interface, and a communication interface.

The modality 30 performs imaging (photographing) according to photographing conditions (photographing protocol) determined on the basis of an image examination instruction of the like, for example. Examples of the modality 30 include an X-ray computed tomography apparatus, an X-ray diagnostic apparatus, a magnetic resonance imaging apparatus, an ultrasonic diagnostic apparatus, a nuclear medicine diagnostic apparatus, and the like. The modality 30 is operated by an operator such as a doctor (radiologist) or a radiological technologist. A medical image (image data) generated by imaging performed by the modality 30 is transmitted to the PACS 40.

The PACS 40 is a computer system that receives medical images transmitted by the modality 30 and stores them in a database. The PACS 40 transmits (transfers) medical images stored in the database in response to requests from clients. The PACS 40 includes a server computer including a processor such as a CPU, memories such as a ROM and a RAM, a display, an input interface, and a communication interface. Information on a patient who is a photographing target and information on photographing are added to medical images stored in the PACS 40 as supplementary information. The supplementary information includes information such as a patient ID, an examination ID, and photographing conditions (photographing protocol) in a format conforming to Digital Imaging and Communication in Medicine (DICOM) standards, for example.

The PACS 40 stores images 41 of a plurality of patients imaged in the past (hereinafter referred to as patient-captured images). The patient-captured images 41 include, for example, an image of each part according to division of the bodies of the patients, for example, images of the chest (lungs and the like), abdomen (stomach, small intestine, and the like), upper limbs, lower limbs (skeleton), and the like. The PACS 40 transmits the stored patient-captured images 41 to the HIS 10 (medical information processing system 100) in response to a request from the HIS 10. The patient-captured images 41 are an example of self-captured images. The patient-captured images 41 stored in the PACS 40 are an example of past captured images (self-captured images) of an examination target.

The configuration of the intra-hospital system 1 is not limited to the aforementioned configuration. The intra-hospital system 1 may include, for example, an image reading report creating device and the like. Further, some elements of the intra-hospital system 1 may be integrated. For example, the HIS 10 and the RIS 20 may be integrated into one system.

FIG. 2 is a block diagram showing an example of a configuration of the medical information processing system 100 of the first embodiment. The medical information processing system 100 includes, for example, a communication interface 110, an input interface 120, a display 130, processing circuitry 140, and a memory 150. The communication interface 110, the input interface 120, and the display 130 in the medical information processing system 100 are provided separately from the communication interface, the input interface, and the display included in the HIS 10, but they may be common.

The communication interface 110 communicates with external devices such as the RIS 20, the modality 30, and the PACS 40, for example, via a network NW such as a local area network (LAN). The communication interface 110 includes, for example, a communication interface such as a network interface card (NIC). The network NW may include the Internet, a cellular network, a Wi-Fi network, a wide area network (WAN), and the like instead of or in addition to the LAN.

The input interface 120 receives various input operations from a medical doctor or the like, converts the received input operations into electrical signals, and outputs the electrical signals to the processing circuitry 140. The input interface 120 generates information according to an input operation, for example, when the input operation is performed by the medical doctor or the like. Input operations for the input interface 120 may include, for example, an operation of inputting information such as an examination purpose, a patient's symptoms, and a medical history. Information such as an examination purpose, a patient's symptoms, and a medical history is useful information by which the structure of the body of a target patient that is different from that in a healthy state can be estimated.

Input operations include, for example, operations regarding generation of order information, selection of reference image candidates, attachment of edited images, and editing of edited images. When the input operations in such cases are executed, the input interface 120 generates generation instruction information, attachment request information, selection information, and image editing information. The input interface 120 outputs generated information according to the input operation to the processing circuitry 140.

The input interface 120 includes, for example, a mouse, a keyboard, a trackball, a switch, a button, a joystick, a touch panel, and the like. The input interface 120 may be, for example, a user interface that receives audio input, such as a microphone. When the input interface 120 is a touch panel, the input interface 120 may also have the display function of the display 130.

The input interface in the specification is not limited to one having physical operation components such as a mouse and a keyboard. For example, examples of the input interface also include electrical signal processing circuitry that receives an electrical signal corresponding to an input operation from external input equipment provided separately from the device and outputs the electrical signal to the control circuit.

The display 130 displays various types of information. For example, the display 130 displays an image generated by the processing circuitry 140, a graphical user interface (GUI) for receiving various input operations from the operator, and the like. For example, the display 130 is a liquid crystal display (LCD), a cathode ray tube (CRT) display, an organic electroluminescence (EL) display, or the like.

The processing circuitry 140 includes, for example, an acquisition function 141, an order generation function 142, an identification function 143, an image generation function 144, and a transmission function 145. The processing circuitry 140 realizes these functions by a hardware processor (computer) executing a program stored in a memory (storage circuit) 150, for example.

The hardware processor refers to, for example, a circuitry such as a CPU, a graphics processing unit (GPU), an application specific integrated circuit (ASIC), a programmable logic device (e.g., a simple programmable logic device (SPLD) or a complex programmable logic device (CPLD), or a field programmable gate array (FPGA).

Instead of storing the program in the memory 150, the program may be directly incorporated into the circuitry of the hardware processor. In this case, the hardware processor realizes the functions by reading and executing the program incorporated into the circuit. The aforementioned program may be stored in the memory 150 in advance, or may be stored in a non-transitory storage medium such as a DVD or CD-ROM and installed in the memory 150 from the non-transitory storage medium by setting the non-transitory storage medium in a drive device (not shown) of the medical information processing system 100.

The hardware processor is not limited to being configured as a single circuit, and may be configured as one hardware processor by combining a plurality of independent circuits to realize each function. Further, a plurality of components may be integrated into one hardware processor to realize each function. The hardware processor, the memory, and the like in the medical information processing system 100 are provided separately from the hardware processor, the memory, and the like of the HIS 10, but they may be common.

The memory 150 stores a plurality of standard reference images 151 and a plurality of reference model images 152. The standard reference images 151 include, for example, an image of each part according to division of the human body, such as images of the chest (lungs and the like), abdomen (stomach, small intestine, and the like), upper limbs, lower limbs (skeleton), and the like. The reference model images 152 include model images that express the human body structure for each part according to division of the human body similar to the reference images. The standard reference images 151 and the reference model images 152 are associated with disease names and medical histories, for example. The standard reference images 151 and the reference model images 152 are examples of past captured images of an examination target.

The memory 150 stores standard reference images for each characteristic. The memory 150 stores, for example, standard reference images 151 for each disease of peoples of different sexes and physiques for each age group, for each part of the human body. The memory 150 stores reference model images 152 for each characteristic similar to the standard reference images.

The standard reference images 151 and the reference model images 152 may be 2D images (two-dimensional images), 3D images (three-dimensional images), or 4D images (four-dimensional images) obtained by adding one dimension, for example, time, to 3D images. The standard reference images 151 may be, for example, CT images or MRI images. The reference model images 152 may be, for example, images generated by computer graphics (CG) or may be images of a human body model.

The acquisition function 141 acquires generation instruction information, attachment request information, selection information, and image editing information transmitted by the input interface 120 in response to input operations performed by a medical doctor or the like. The acquisition function 141 acquires information about a patient who is an examination target (hereinafter, a target patient), for example, patient information. The acquisition function 141 acquires the patient-captured images 41 transmitted by the PACS 40. The acquisition function 141 reads and acquires the standard reference images 151 and the reference model images 152 stored in the memory 150. The acquisition function 141 is an example of an acquisition unit.

The order generation function 142 starts generation of order information when the generation instruction information transmitted by the input interface 120 is acquired by the acquisition function 141. The generation instruction information is transmitted at an arbitrary timing by a medical doctor or the like. The generation instruction information is transmitted, for example, when patient information is displayed on the display of the HIS 10.

The order generation function 142 edits and generates order information on the basis of the generation instruction information, the patient information, and the like acquired by the acquisition function 141. The order information includes examination purpose information indicating the content of an examination to be ordered. The examination purpose information includes, for example, text information. The examination purpose information includes, for example, an examination purpose, patient's symptoms, a patient's medical history, and the like. The examination purpose information may be text written by the medical doctor or the like, or may be text of which a part or all is standard wording or is selected by an option. The examination purpose information generated here includes patient information of the target patient.

When an edited image is generated by the image generation function 144, the order generation function 142 attaches the generated edited image to the order information. After the edited image is attached to the order information, the image generation function 144 re-edits the examination purpose information on the basis of transmitted generation instruction information when the generation instruction information is transmitted through the input interface 120 in response to an input operation performed by the medical doctor or the like. The order generation function 142 may attach the edited image to the order information in advance before generating the examination purpose information.

When the acquisition function 141 acquires attachment request information, the identification function 143 causes the acquisition function 141 to acquire reference image candidates based on the patient information acquired by the acquisition function 141. The patient information may be information included in the examination purpose information or may be information transmitted by the electronic medical record system 11. The reference image candidates are at least one of patient-captured images 41, standard reference images 151, and reference model images 152, for example. The identification function 143 causes the acquisition function 141 to acquire selected reference image candidates.

The identification function 143 causes the display 130 to display the reference image candidates acquired by the acquisition function 141. The identification function 143 identifies a reference image corresponding to the target patient from among the reference image candidates acquired by the acquisition function 141. The identification function 143 selects the reference image from the reference image candidates on the basis of selection information acquired by the acquisition function 141.

The identification function 143 may identify the reference image, for example, on the basis of the patient information, particularly the characteristics of the target patient included in the patient information. In this case, the identification function 143 causes the acquisition function 141 to acquire the identified reference image without selecting reference image candidates. If standard reference images 151 and reference model images 152 are not stored in the memory 150 for each characteristic, the identification function 143 may identify a reference image on the basis of information corresponding to the patient information. The identification function 143 may identify one reference image or a plurality of reference images. The identification function 143 is an example of an identification unit.

The image generation function 144 generates an edited image by editing the reference image identified by the identification function 143 on the basis of image editing information acquired by the acquisition function 141. The edited image is an image attached to the examination purpose information in order to assist the examination purpose indicated by text information. The medical doctor or the like explains instructions that are difficult to deliver or that cannot be delivered by text information, for example, using the edited image subsidiarily.

For example, the image generation function 144 generates an edited image by changing the imaging direction of the reference image. For example, the image generation function 144 generates an edited image by changing the imaging range of the reference image. The image generation function 144 may generate an edited image by editing the reference image on the basis of the patient information or other information, or may generate an edited image by editing the reference image on the basis of the image editing information acquired by the acquisition function 141. The image generation function 144 is an example of a generation unit.

For example, the image generation function 144 refers to text information in the examination purpose information of the order information and automatically generates the edited image. The image generation function 144 determines a target portion (reference portion in the edited image) based on information on an examination purpose, patient's symptoms, an imaging target portion, a disease, and the like. After determining the target portion, the image generation function 144 detects the target portion in the reference image using a conventional method (for example, anatomical landmark detection).

The image generation function 144 determines a display setting based on the target portion to make it easier to observe the target portion in the reference image, i.e., to increase visibility of the target portion. The image generation function 144 determines the display setting, for example, (i) changing of a display range, (ii) changing of a display direction, (iii) deleting of an unrelated portion, and (iv) changing of an image filter. When (i) changing of a display range, the image generation function 144 determines the display range so that the entire of the target portion is included in the display range and the display area of the target portion is maximized, i.e., the target portion in the original reference image is enlarged. When (ii) changing of a display direction, the image generation function 144 determines the display direction so that the display area of the target portion is maximized or the edge amount of the target portion is maximized. When (iii) deleting of an unrelated portion, the image generation function 144 deletes a region other than the target portion determined based on the text information or deletes portions other than any portions related to the target portion and a target disease. When (iv) changing of an image filter, the image generation function 144 selects an image filter based on the examination purpose and/or the target portion. In this case, the image filter may be determined using a color lookup table (for example, a lookup table LTU) based on the combination of the text information indicating an examination purpose or the like and the target portion.

The transmission function 145 transmits the order information generated by the order generation function 142 to the RIS 20 using the communication interface 110. When an edited image is attached to the order information and the examination purpose information and the edited image are included therein, the transmission function 145 transmits the order information including the examination purpose information and the edited image to the RIS 20. The transmission function 145 is an example of a transmission unit.

Although the transmission function 145 transmits, to the RIS 20, only the examination purpose information or the order information including the examination purpose information and the edited image, the transmission function 145 may transmit the order information and the like in another manner. For example, the transmission function 145 may link the examination purpose information and the edited image, and then individually transmit the examination purpose information and the edited image to the RIS 20. Alternatively, the transmission function 145 may transmit the examination purpose information to the RIS 20, attach an image data ID to the edited image, and transmit the edited image to the PACS 40. In this case, the transmission function 145 may attach the image data ID attached to the edited image to the examination purpose information and transmit it to the RIS 20 such that the RIS 20 can acquire the edited image from the PACS 40.

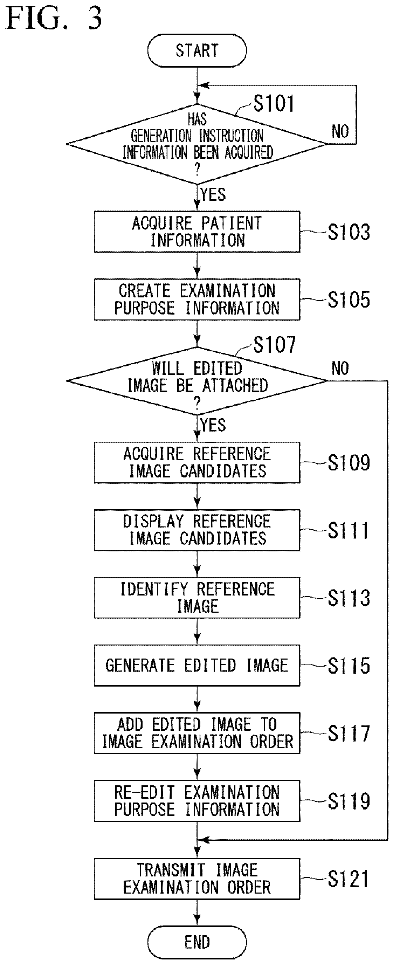

Next, processing in the medical information processing system 100 will be described. FIG. 3 is a flowchart showing an example of processing in the medical information processing system 100 of the first embodiment. The medical information processing system 100 determines whether or not generation instruction information transmitted through the input interface 120 has been acquired (step S 101). If it is determined that the generation instruction information has not been acquired, the medical information processing system 100 repeats processing of step S101.

If it is determined that the generation instruction information has been acquired, the medical information processing system 100 acquires patient information transmitted by the electronic medical record system 11 through the acquisition function 141 (step S103). Subsequently, the order generation function 142 generates examination purpose information indicating a purpose of an image examination order on the basis of the generation instruction information and the patient information acquired by the acquisition function 141 (step S105).

Subsequently, the order generation function 142 determines whether or not an edited image will be attached to order information on the basis of whether or not the acquisition function 141 has acquired attachment request information transmitted through the input interface 120 (step S107). If the acquisition function 141 has not acquired the attachment request information and it is determined that an edited image will not be attached, the order generation function 142 proceeds to step S121.

If the attachment request information has been acquired and it is determined that an edited image will be attached, the acquisition function 141 acquires a plurality of reference image candidates on the basis of the acquired patient information (step S109). The acquisition function 141 transmits characteristics included in the patient information to the PACS 40 in order to acquire the reference image candidates. The PACS 40 transmits patient-captured images 41 corresponding to the characteristics included in the patient information. The acquisition function 141 reads and acquires standard reference images 151 and reference model images 152 corresponding to the characteristics included in the patient information.

The identification function 143 causes the display 130 to display the patient-captured images 41, the standard reference images 151, and the reference model images 152 acquired by the acquisition function 141 as reference image candidates (step S111). For example, when the patient-captured images 41 corresponding to the patient ID included in the patient information is stored in the PACS 40, the identification function 143 causes the acquisition function 141 to acquire the patient-captured images as reference image candidates and causes the display 130 to display them.

For example, if the patient information includes a suspected disease name, the display 130 displays images associated with the same disease as the suspected disease name and images associated with differential diseases (other diseases that need to be differentiated to exhibit the same symptoms as the suspected disease) from among the patient-captured images 41, the standard reference images 151, and the reference model images 152 as reference image candidates. For example, if the patient information includes a medical history, images associated with the same disease as a disease name in the medical history from among the patient-captured images 41, the standard reference images 151, and the reference model images 152 are displayed as reference image candidates.

The medical doctor or the like who views display 130 performs an input operation for designating one or more reference image candidates that can be used to generate an edited image on the input interface 120. The input interface 120 outputs selection information according to the input operation to the processing circuitry 140. The acquisition function 141 acquires the selection information transmitted by the input interface 120.

Subsequently, the identification function 143 selects one or a plurality of reference image candidates designated by the medical doctor or the like from the reference image candidates on the basis of the selection information output by the input interface 120 and acquired by the acquisition function 141. The identification function 143 identifies the selected reference image candidates as reference images (step S113).

Subsequently, the image generation function 144 edits the reference images identified by the identification function 143 to generate edited images (step S115). Processing for generating an edited image will be further described later. Subsequently, the order generation function 142 attaches the edited images to the order information (step S117).

Subsequently, the order generation function 142 causes the display 130 to display the examination purpose information and the edited images included in the image examination order. The medical doctor or the like performs an input operation for reconstructing text of the examination purpose information on the input interface 120 while viewing the display of the display 130. The input interface 120 outputs generation instruction information according to the input operation to the processing circuitry 140. The acquisition function 141 acquires the generation instruction information transmitted by the input interface 120.

Subsequently, the order generation function 142 re-edits the examination purpose information on the basis of the generation instruction information acquired by the acquisition function 141 (step S119). Subsequently, the transmission function 145 transmits the image examination order generated or re-edited by the order generation function 142 to the RIS 20 (step S121). In this manner, the medical information processing system 100 ends processing shown in FIG. 3.

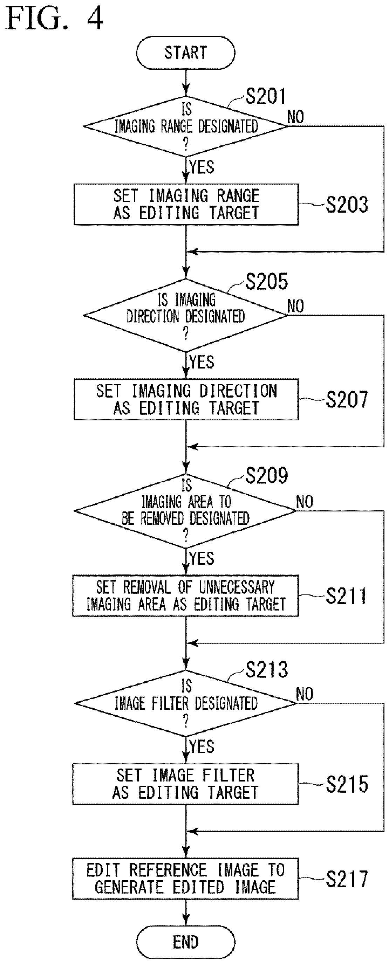

Next, processing for generating an edited image will be described. FIG. 4 is a flowchart showing an example of processing in the medical information processing system 100. FIG. 4 illustrates processing of the medical information processing system 100 focusing on processing for generating an edited image. In generating an edited image, the order generation function 142 edits and generates the edited image according to generation instruction information acquired by the acquisition function 141.

The order generation function 142 determines whether or not the generation instruction information acquired by the acquisition function 141 indicates an instruction to designate an imaging range in order to change the imaging range (step S201). When it is determined that the generation instruction information indicates the instruction to designate the imaging range, the order generation function 142 sets the imaging range as spatial information as an editing target, for example (step S203). If it is determined that the generation instruction information indicates an instruction not to designate the imaging range, the order generation function 142 skips processing of step S203.

Subsequently, the order generation function 142 determines whether or not the generation instruction information acquired by the acquisition function 141 indicates an instruction to designate an imaging direction in order to change the imaging direction (step S205). When it is determined that the generation instruction information indicates the instruction to designate the imaging direction, the order generation function 142 sets the imaging direction as spatial information as an editing target, for example (step S207). When it is determined that the generation instruction information indicates an instruction not to designate the imaging direction, the order generation function 142 skips processing of step S207.

Subsequently, the order generation function 142 determines whether or not the generation instruction information acquired by the acquisition function 141 indicates an instruction to designate an imaging area to be removed (step S209). When it is determined that the generation instruction information indicates the instruction to designate the imaging area to be removed, the order generation function 142 sets removal of the unnecessary imaging area as an editing target (step S211). When it is determined that the generation instruction information indicates an instruction not to designate an imaging area to be removed, the order generation function 142 skips processing of step S211.

Subsequently, the order generation function 142 determines whether or not the generation instruction information acquired by the acquisition function 141 indicates an instruction to designate an image filter (step S213). When it is determined that the generation instruction information indicates the instruction to designate an image filter, the order generation function 142 sets the image filter as an editing target (step S215). When it is determined that the generation instruction information indicates an instruction not to designate an image filter, the order generation function 142 skips processing of step S215.

Subsequently, the order generation function 142 generates an edited image by editing a reference image on the basis of the generation instruction information acquired by the acquisition function 141 with respect to an item set as an editing target (step S217). In this manner, the medical information processing system 100 ends processing shown in FIG. 4, and proceeds to step S117 in FIG. 3.

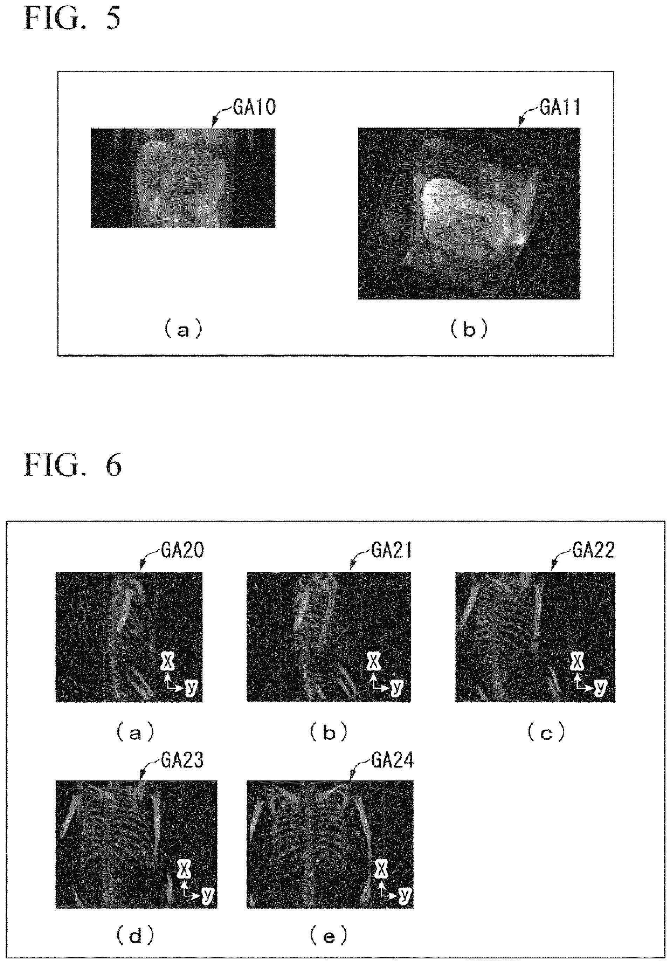

Next, a first example of editing a reference image to generate an edited image will be described. FIG. 5 is a diagram showing an example of a reference image GA10 and an edited image GA11. The reference image GA10 shown in (a) of FIG. 5 is a 3D abdominal contrast-enhanced MRI image. In the first example, an image examination order focusing on a specific hepatic artery is issued.

Generation instruction information in the first example is information including an instruction to look at the specific hepatic artery. The edited image GA11 shown in (b) of FIG. 5 is an image adjusted by changing the range and direction of the reference image GA10 and edited so as to become an image focused on the hepatic artery. In this manner, the order generation function 142 generates the edited image GA11 according to the generation instruction information.

Next, a second example of editing a reference image to generate an edited image will be described. FIG. 6 is a diagram showing an example of a reference image GA20 and a first edited image GA21 to a fourth edited image GA24. The reference image GA20 shown in (a) of FIG. 6 and the first edited image GA21 to the fourth edited image GA24 shown in (b) to (e) of FIG. 6 are all 3D chest CT model images. In the second example, an image examination order for looking at the chest of a patient in a specific direction is issued.

Generation instruction information in the second example is information including an instruction to look at the chest of the patient in a specific direction. In generating an edited image, the order generation function 142 rotates the 3D chest CT model image of the reference image GA20 and causes the display 130 to sequentially display images in different viewing directions. A medical doctor or the like selects any one of the first edited image GA21 to the fourth edited image GA24 shown in (b) to (e) of FIG. 6 from among the 3D chest CT model images sequentially displayed on the display 130. The order generation function 142 generates the image selected by the medical doctor or the like as an edited image.

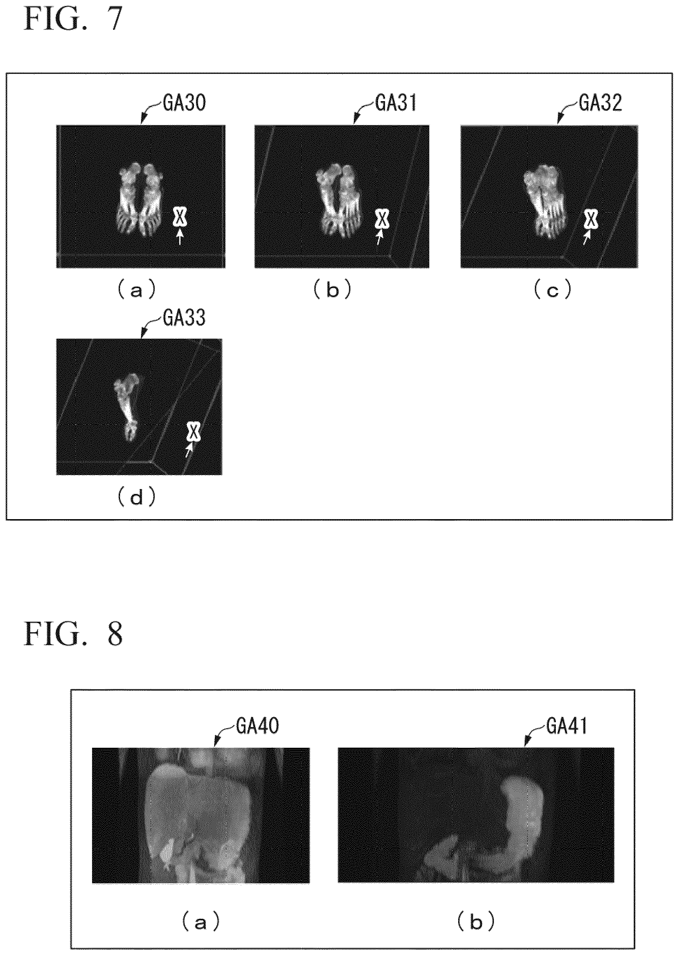

Next, a third example of editing a reference image to generate an edited image will be described. FIG. 7 is a diagram showing an example of a reference image GA30 and a first edited image GA31 to a third edited image GA33. The reference image GA30 shown in (a) of FIG. 7 is a 3D foot CT image. In the third example, for example, there is a fracture in a part of the right foot, and an image examination order focusing on the right foot viewed in a specific direction is issued.

Generation instruction information in the third example is information including an instruction to look at the feet of a patient in a specific direction. In generating an edited image, the order generation function 142 rotates the 3D foot CT image of the reference image GA30 and causes the display 130 to sequentially display images in different viewing directions. The medical doctor or the like selects any one of the first edited image GA31 to the third edited image GA33 shown in (b) to (d) of FIG. 7 from among the 3D foot CT images sequentially displayed on the display 130. The order generation function 142 generates an image selected by the medical doctor or the like as an edited image.

Next, a fourth example of editing a reference image to generate an edited image will be described. FIG. 8 is a diagram showing an example of a reference image GA40 and an edited image GA41. In the fourth example, for example, an image examination order focusing on the stomach is issued.

Generation instruction information in the fourth example is information including an instruction to look at the stomach of a patient. In generating an edited image, the order generation function 142 causes the display 130 to display the reference image GA40 shown in (a) of FIG. 8. In order to make the stomach visible, the order generation function 142 removes an area where organs other than the stomach are displayed and generates an image edited such that the stomach can be easily viewed. In this manner, the order generation function 142 generates the edited image GA41 shown in (b) of FIG. 8 according to the generation instruction information.

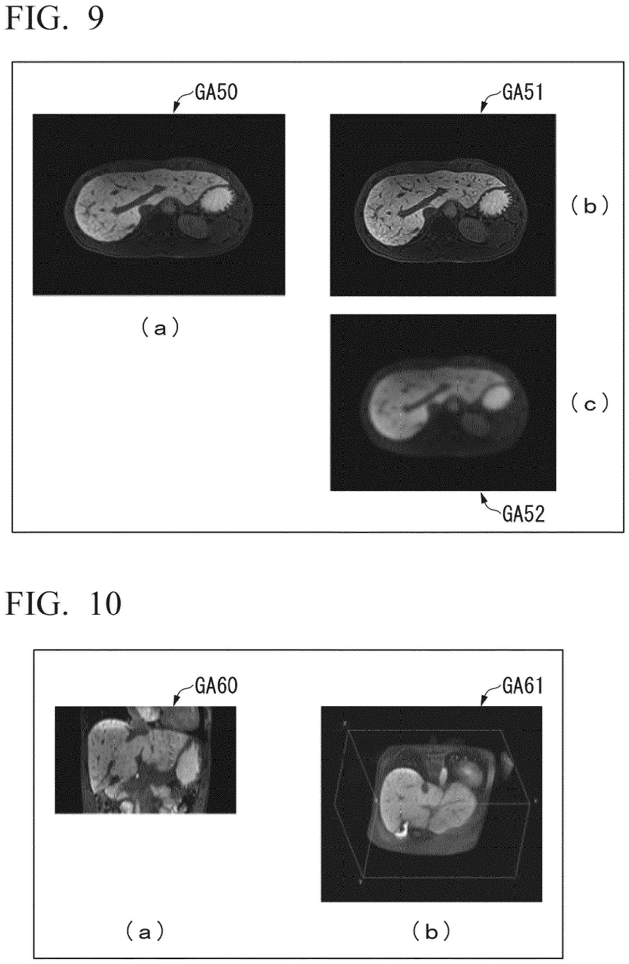

Next, a fifth example of editing a reference image to generate an edited image will be described. FIG. 9 is a diagram showing an example of a reference image GA50, a first edited image GA51, and a second edited image GA52. The reference image GA50 shown in (a) of FIG. 9 is a 3D abdominal contrast-enhanced MRI image. In the fifth example, generation instruction information designates information on the appearance of an image. The information on the appearance of an image includes, for example, a degree of definition of the image and final image information.

Generation instruction information in the fifth example designates, for example, an image filter such as a sharpness image filter or a smoothing image filter to be applied to the reference image and designates a degree of definition of the image necessary for an examination. The first edited image GA51 shown in (b) of FIG. 9 is an image edited by performing sharpness processing on the reference image GA50. The second edited image GA52 shown in (c) of FIG. 9 is an image edited by performing smoothing processing on the reference image GA50.

In general, the higher the definition of imaging, the longer the imaging time and the higher the cost. On the other hand, the degree of definition required to achieve an examination purpose may not necessarily be high. For example, an examination purpose may be to check that the entire liver is significantly contrast-enhanced and the hepatic artery does not have a severe blood flow disorder. In order to achieve this examination purpose, it is sufficient to obtain an image by which the fact that the entire liver exhibits a high signal by contrast enhancement can be checked, and a highdefinition image that shows fine vasculature is not necessary. Therefore, in such a case, the cost can be reduced by generating the second edited image GA52 edited by performing smoothing processing on the reference image GA50.

Next, a sixth example of editing a reference image to generate an edited image will be described. FIG. 10 is a diagram showing an example of a reference image GA60 and an edited image GA61. The reference image GA60 shown in (a) of FIG. 10 is a 3D abdominal contrast-enhanced MRI image. In the sixth example, generation instruction information is information for instructing a final image designating volume rendering processing in addition to instruction for a range and a direction in the first example.

The order generation function 142 executes volume rendering processing on the basis of the generation instruction information to perform editing in which the appearance of an image is three-dimensionally processed to generate the edited image GA61 shown in (b) of FIG. 10. By performing such processing, it is possible to improve the degree of understanding of the patient at the time of explaining the patient's condition and the like and to aid in consideration of operation techniques at the time of generating an operation plan.

In each of the aforementioned examples, at the time of editing a reference image to generate an edited image, the order generation function 142 edits the image itself, but editing may be performed by adding an arrow mark, a circle mark, or the like to the image before or after editing. For example, when there is a part focused as an examination target, the order generation function 142 may enclose the part with a circle, add a symbol indicated by an arrow, and describe the content with text information. Further, although an edited image is generated on the basis of image editing information according to an input operation performed by a medical doctor or the like, an edited image may be generated on the basis of information other than the image editing information according to the input operation performed by the medical doctor or the like. An edited image may be generated according to, for example, the symptoms and a degree of progress of a lesion.

### <Second embodiment>

A second embodiment will be described below. The second embodiment differs from the above-described first embodiment in that history information regarding operations performed on reference images at the time of generating edited images and explanatory information for delivering the intention of editing are added to an image examination order. Hereinafter, differences from the first embodiment will be mainly described, and description of common points with respect to the first embodiment will be omitted. In description of the second embodiment, parts that are the same as in the first embodiment will be denoted by the same reference numerals and described.

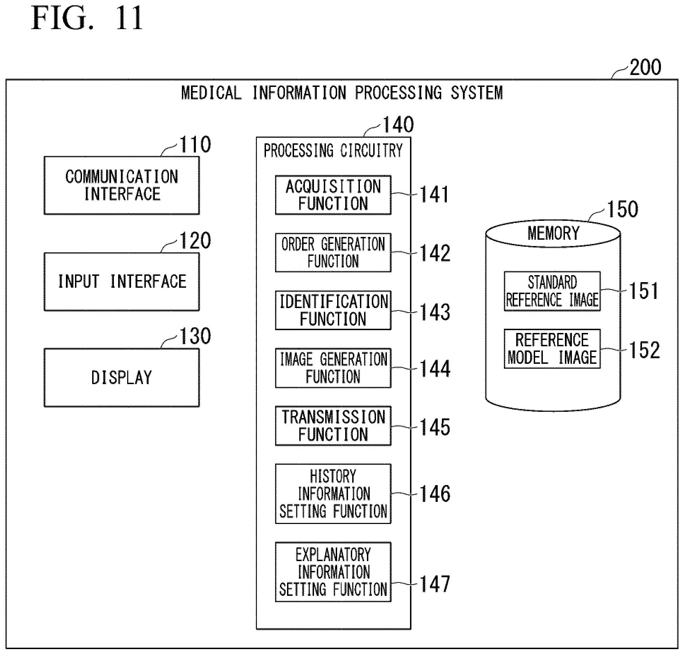

FIG. 11 is a block diagram showing an example of a configuration of a medical information processing system 200 of the second embodiment. Compared to the medical information processing system 100 of the first embodiment, the processing circuitry 140 of the medical information processing system 200 further includes a history information setting function 146 and an explanatory information setting function 147.

The history information setting function 146 acquires history information regarding an operation when the operation has been performed on a reference image on the basis of selection information, image editing information, and the like transmitted through the input interface 120 according to input operation performed by a user such as a medical doctor. The time when an operation has been performed on a reference image is, for example, all or part of the period from acquisition of reference image candidates to generation of an edited image. The history information setting function 146 acquires, as history information, for example, moving image information representing transitions of screens displayed on the display 130 when an operation is performed on a reference image. In addition, the history information setting function 146 acquires, as history information, text information indicating various operation events of generation instruction information, attachment request information, selection information, and image editing information when an operation is performed on a reference image, for example. By using such history information, it is possible to ascertain which reference image candidate has been selected by the user and what kind of editing processing has been performed on the selected reference image (content of editing processing, order, and the like). The history information setting function 146 is an example of a history information setting unit. The history information setting function 146 sets history information indicating a history of operations performed on a reference image.

After an edited image is generated, the explanatory information setting function 147 sets caption information of the edited image (description attached to the edited image) and annotation information of the edited image (character strings displayed on the edited image, or arrows and handwritten lines displayed on the edited image) on the basis of explanatory information transmitted through the input interface 120 according to input operation performed by the user such as a medical doctor. The explanatory information setting function 147 is an example of an explanatory information setting unit. The explanatory information setting function 147 sets explanatory information for the edited image.

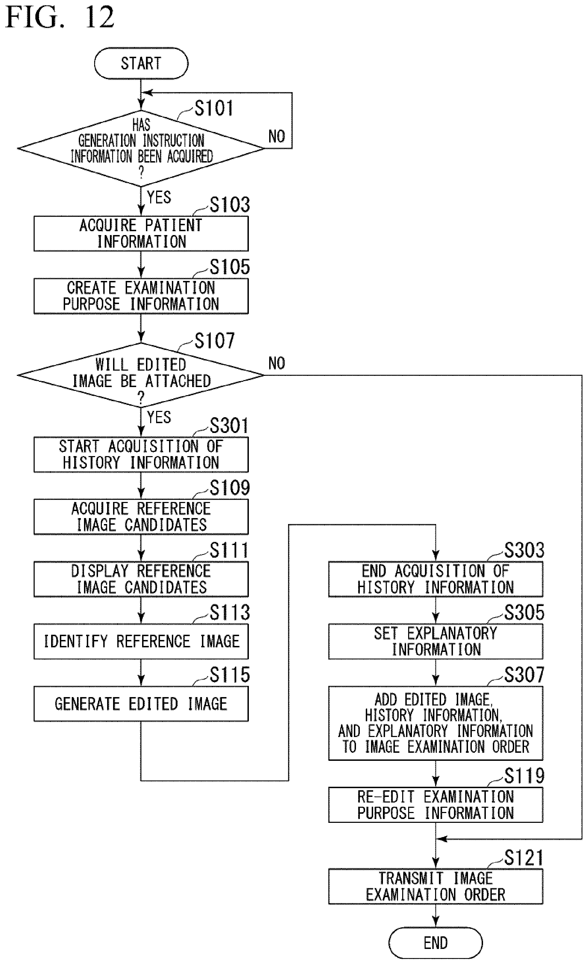

Next, processing in the medical information processing system 200 will be described. FIG. 12 is a flowchart showing an example of processing in the medical information processing system 200 of the second embodiment. The medical information processing system 200 determines whether or not generation instruction information transmitted by the input interface 120 has been acquired (step S101). When it is determined that the generation instruction information has not been acquired, the medical information processing system 200 repeats processing of step S101.

When it is determined that the generation instruction information has been acquired, the medical information processing system 200 acquires patient information transmitted by the electronic medical record system 11 through the acquisition function 141 (step S103). Subsequently, the order generation function 142 generates examination purpose information indicating a purpose of an image examination order on the basis of the generation instruction information and the patient information acquired by the acquisition function 141 (step S105).

Subsequently, the order generation function 142 determines whether or not to attach an edited image to order information on the basis of whether or not the acquisition function 141 has acquired attachment request information transmitted through the input interface 120 (step S107). If the acquisition function 141 has not acquired the attachment request information and it is determined that an edited image will not be attached, the order generation function 142 proceeds to step S121.

When it is determined that the attachment request information has been acquired and the edited image will be attached, the history information setting function 146 starts acquisition of history information regarding operations performed on reference images (step S301).

Next, the acquisition function 141 acquires a plurality of reference image candidates on the basis of the acquired patient information (step S109). The identification function 143 causes the display 130 to display patient-captured images 41, standard reference images 151, and reference model images 152 acquired by the acquisition function 141 as reference image candidates (step Sill).

Subsequently, the identification function 143 selects one or a plurality of reference image candidates designated by a medical doctor or the like from among the reference image candidates on the basis of selection information output by the input interface 120 and acquired by the acquisition function 141. The identification function 143 identifies the selected reference image candidate as a reference image (step S113). Subsequently, the image generation function 144 edits the reference image identified by the identification function 143 to generate an edited image (step S115).

Next, the history information setting function 146 ends acquisition of the history information (step S303). Further, the explanatory information setting function 147 sets explanatory information transmitted through the input interface 120 according to an input operation performed by the user such as a medical doctor in the edited image (step S305).

Next, the order generation function 142 attaches the edited image, the history information, and the explanatory information to the order information (step S307).

Subsequently, the order generation function 142 causes the display 130 to display the examination purpose information, the edited image, the history information, and the explanatory information included in the image examination order. The medical doctor or the like executes an input operation for reconstructing text of the examination purpose information on the input interface 120 while viewing the display of the display 130. The input interface 120 outputs generation instruction information according to the input operation to the processing circuitry 140. The acquisition function 141 acquires the generation instruction information transmitted by the input interface 120.

Subsequently, the order generation function 142 re-edits the examination purpose information on the basis of the generation instruction information acquired by the acquisition function 141 (step S119). Subsequently, the transmission function 145 transmits the image examination order generated or re-edited by the order generation function 142 to the RIS 20 (step S121). In this manner, the medical information processing system 200 ends processing shown in FIG. 12.

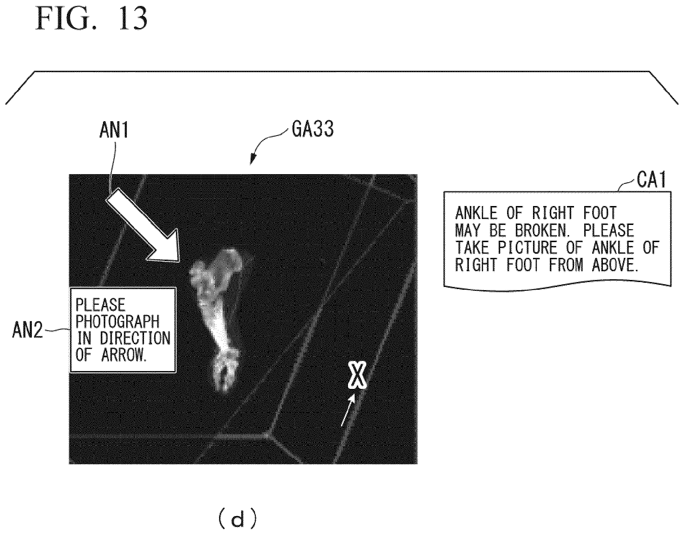

FIG. 13 is a diagram showing a state in which explanatory information has been added to the third edited image GA33. In the example shown in FIG. 13, a comment "The ankle of the right foot may be broken. Please take a picture of the ankle of the right foot from above." as caption information CA1 is attached to the third edited image GA33 shown in (d) of FIG. 7. Furthermore, an arrow indicating the photographing direction as annotation information AN1 and a character string "Please shoot in the direction of the arrow" as annotation information AN2 are attached to the third edited image GA33. In this manner, by adding explanatory information to the edited image, it is possible to clarify the intention of the editing operation performed by the medical doctor or the like on the image examination order.

According to at least one embodiment described above, it is possible to accurately and easily deliver instructions regarding an examination by including an acquisition unit that acquires information on an examination target, an identification unit that identifies a reference image corresponding to the examination target on the basis of the information on the examination target, an image generation unit that generates an edited image generated by editing the reference image, and a transmission unit that transmits order information to which the edited image has been attached.

Although several embodiments have been described, these embodiments are presented as examples and are not intended to limit the scope of the invention. These embodiments can be implemented in various other forms, and various omissions, substitutions, and modifications can be made without departing from the scope of the invention as defined be the appended claims.

## Claims

1. A medical information processing system comprising:
an acquisition unit (141) configured to acquire information on an examination target;
an identification unit (143) configured to identify a reference image (151) corresponding to the examination target on the basis of the information on the examination target;
an image generation unit (144) configured to generate an edited image generated by editing the reference image (151); and
a transmission unit (145) configured to transmit order information to which the edited image has been attached.

2. The medical information processing system according to claim 1, wherein the acquisition unit (141) is configured to further acquire patient information on a target patient, and the identification unit (143) is configured to identify the reference image (151) on the basis of the information on the examination target and the patient information.

3. The medical information processing system according to claim 1, wherein the identification unit (143) is configured to identify the reference image (151) from among previously captured images of the examination target.

4. The medical information processing system according to claim 2, wherein the reference image (151) is identified from among past self-captured images of the examination target in the target patient.

5. The medical information processing system according to claim 2 or 4, wherein the identification unit (143) is configured to identify the reference image (151) according to characteristics of the target patient included in the patient information.

6. The medical information processing system according to claim 2 or 4, wherein the identification unit (143) is configured to identify the reference image (151) from among model images (152) expressing a human body structure according to the characteristics of the target patient.

7. The medical information processing system according to claim 5 or 6, wherein the characteristics of the target patient include at least one of age, sex, physique, or diseases.

8. The medical information processing system according to any one of claims 1 to 7, wherein the identification unit (143) is configured to identify the reference image (151) on the basis of selection information transmitted through an input interface operated by an operator.

9. The medical information processing system according to any one of claims 1 to 8, wherein the image generation unit (144) is configured to generate the edited image by changing an imaging direction of the reference image (151).

10. The medical information processing system according to any one of claims 1 to 9, wherein the image generation unit (144) is configured to generate the edited image obtained by changing an imaging range of the reference image (151).

11. The medical information processing system according to any one of claims 1 to 10, wherein the image generation unit (144) is configured to generate the edited image by applying an image filter to the reference image (151).

12. The medical information processing system according to any one of claims 1 to 11, wherein the image generation unit (144) is configured to generate the edited image obtained by removing an area of a part of the imaging range of the reference image (151).

13. The medical information processing system according to any one of claims 1 to 12, further comprising a history information setting unit (146) configured to set history information indicating a history of operations performed on the reference image,
wherein the transmission unit (145) is configured to transmit the order information to which the edited image and the history information have been attached.

14. The medical information processing system according to any one of claims 1 to 13, further comprising an explanatory information setting unit (147) configured to set explanatory information for the edited image,
wherein the transmission unit (145) is configured to transmit the order information to which the edited image for which the explanatory information has been set has been attached.

15. The medical information processing system according to any one of claims 1 to 14, wherein the image generation unit (144) is configured to:
determine a target portion based on text information in examination purpose information;
detect the target portion in the reference image; and
generate the edited image based on the detected target portion.

16. A medical information processing method, performed by a computer, comprising:
acquiring information on an examination target;
identifying a reference image corresponding to the examination target on the basis of the information on the examination target;
generating an edited image generated by editing the reference image; and
transmitting order information to which the edited image has been attached.

17. A program causing a computer to:
acquire information on an examination target;
identify a reference image corresponding to the examination target on the basis of the information on the examination target;
generate an edited image generated by editing the reference image; and
transmit order information to which the edited image has been attached.
